(19) Europäisches Patentamt
European Patent Office
Office européen des brevets

(11) **EP 4 299 809 A1**

(12) # EUROPEAN PATENT APPLICATION

(43) Date of publication:
**03.01.2024 Bulletin 2024/01**

(21) Application number: **22204635.1**

(22) Date of filing: **31.10.2022**

(51) International Patent Classification (IPC):
**D04H 1/425** (2012.01)    **D04H 1/4258** (2012.01)
**D04H 1/4391** (2012.01)    **D04H 1/74** (2006.01)
**A61F 13/537** (2006.01)    **A61K 8/02** (2006.01)

(52) Cooperative Patent Classification (CPC):
**D04H 1/425; D04H 1/4258; D04H 1/4391;
D04H 1/43912; D04H 1/43918; D04H 1/498;
D04H 1/74;** A61F 13/537; A61K 8/0208

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Lenzing Aktiengesellschaft
4860 Lenzing (AT)**

(72) Inventors:
- **Porcher, Alexis
  33590 Saint-Vivien de Médoc (FR)**
- **Maier, Thomas
  4863 Seewalchen (AT)**

(74) Representative: **Global Patent Management
Lenzing AG
Werkstraße 2
4860 Lenzing (AT)**

(54)   **NONWOVEN SUBSTRATE COMPRISING AT LEAST ONE FIBROUS LAYER**

(57)   Nonwoven substrate comprising at least one fibrous layer formed by a dry web-formation technique. The fibrous layer comprises from 10 % w/w to 90 % w/w plain fibers, preferably from 20% w/w to 80% w/w plain fibers, and from 10 % w/w to 90 % w/w stiffening fibers, preferably from 20% w/w to 80 % w/w stiffening fibers.

The plain fibers are regenerated cellulosic fibers with a titer of between 1.2 and 1.9 dtex and the stiffening fibers are natural or regenerated cellulosic fibers with a stiffening cross-section. At least one of the plain fibers and/or the stiffening fibers are provided with a mechanical crimp or a natural crimp.

Fig. 1

EP 4 299 809 A1

**Description**

**Field of the invention**

The invention relates to a nonwoven substrate comprising at least one fibrous layer formed by a dry web-formation technique.

**Description of the Related Art**

Especially for the production of wet wipes and hygiene towels, but also in many other fields of use, nonwoven fabrics are required that combine a low basis weight with high tensile strength. Also, many products should be biodegradable and/or flushable, which often precludes the use of cheap and strong plastic fibers. For these and other reasons, regenerated cellulosic fibers are increasingly used for the production of nonwoven substrates. Nonetheless, without the use of environmentally problematic synthetic fibers it is difficult to reduce the basis weight of the nonwoven substrate while providing the required tensile strength.

Further, especially nonwoven substrates that shall be useable for the production of wet-wipes should keep a stable form even when wet, as they are used in a wet state. Many nonwoven products tend to "collapse" when wetted, and loose their favorable properties.

WO2021/197927 A1 discloses nonwoven substrate containing a mixture of at least three different fiber types, namely lyocell fibers with a titer of between 1.5 and 1.9 dtex, viscose fibers with a titer of between 1.5 and 1.9 dtex and cellulosic man made fibers with a stiffening cross-section and with a cut length of between 30 and 70 mm. The nonwoven substrate can consist of purely cellulosic fibers.

It is an objective of the present disclosure to provide methods and products that can be used for the production of improved nonwoven products. Especially nonwoven substrates shall be provided that combine a low basis weight with a high tensile strength and a low wet collapse, while preferably being 100 % cellulosic.

**Summary**

The present disclosure relates to a nonwoven substrate comprising at least one fibrous layer formed by a dry web-formation technique, the fibrous layer comprising from 10 % w/w to 90 % w/w plain fibers, preferably from 20% w/w to 80% w/w plain fibers, and from 10 % w/w to 90 % w/w stiffening fibers, preferably from 20% w/w to 80 % w/w stiffening fibers, wherein the plain fibers are regenerated cellulosic fibers with a titer of between 1.2 and 1.9 dtex and the stiffening fibers are natural or regenerated cellulosic fibers with a stiffening cross-section, wherein at least one of the plain fibers and/or the stiffening fibers being provided with a mechanical crimp or a natural crimp.

It has been found that by the addition of stiffening fibers to plain fibers according to the features disclosed herein, a surprisingly good wet stability can be reached in a reproducible manner.

The term "regenerated cellulosic fibers", as it is used herein, denotes man-made cellulosic fibers that are based on cellulosic matter as a source material.

In the context of the current disclosure the term "cellulose" denotes an organic compound derived from plant cell walls or synthetically produced. Cellulose is a polysaccharide and is unbranched. Typically, cellulose comprises several hundred to ten thousand β-D-glucose molecules (β-1,4-glycosidic bound) or cellobiose units, respectively. The cellulose molecules that are used by plants to produce cellulose fibers are also used in technical processes to produce regenerated cellulose.

The term "regenerated cellulose" denotes a class of materials manufactured by the conversion of natural or recycled cellulose to a soluble cellulosic derivative or a directly dissolved cellulose solution and subsequent regeneration, forming shaped bodies, such as fibers (e.g., rayon), films or foils (e.g., cellophane) or bulk solids (e.g. beads, powders or pellets).

The term "natural cellulosic fibers", as it is used herein, denotes fibers directly derived from plants. Natural cellulosic fibers, and especially the cellulose in these fibers, keep a structure as grown in the plant. Examples for natural cellulosic fibers include, jute, flax, hemp, ramie, sisal, coir and the like.

The term "fibers", as it is used herein, denotes cut staple fibers of any desired length.

The term "nonwoven substrate" denotes an assembly of textile fibers held together by mechanical interlocking in a random web or mat.

As "viscose fibers", regenerated cellulosic fibers are denoted, which are manufactured by means of a wet spinning method which is called viscose-method.

The starting raw material of the viscose-method is cellulose which is usually provided on the basis of wood. From this starting raw material a highly pure cellulose in form of chemical pulp is obtained. Additionally or as an alternative other cellulosic materials, such as bamboo, cotton linters, recycled cellulosic materials, reed, etc., or mixtures of such materials can be used as a starting raw material. In subsequent process stages, the pulp is first treated with caustic soda (NaOH), whereby alkali cellulose is formed. In a subsequent conversion of said alkali cellulose with carbon disulfide, cellulose-xanthogenate is formed. From this, by further supplying NaOH, the viscose-spinning solution is generated which is pumped through holes of shower-like spinning nozzles into a coagulation bath (also referred to as spin bath). There, one viscose-filament per spinning nozzle hole is generated by coagulation. To coagulate the spinning solution, an acidic coagulation bath is used. The thus generated viscose-filaments are subsequently post processed. The post processing usually comprises several washing- and stretching steps and the filaments are cut to viscose-staple fibers. Several other post-processing steps, such as crimping, bleaching and/or finishing ("soft finish") can be performed on the uncut and/or the cut fibers. Another well-known process for the manufacturing of regenerated cellulose fibers is the carbamate-method, which is similar to the viscose-process but uses urea instead of carbon disulfide. Still another process, which is called modal-process, is a modified viscose-process for the production of higher quality fibers. In the context of this document, the term "viscose process" denotes any of these processes.

The term "lyocell", as used herein, denotes a regenerated fiber type comprising cellulose, which is manufactured according to a direct solvent method. The cellulose for the lyocell-method is extracted from the raw material containing the cellulose. The thus obtained pulp may subsequently be dissolved in a suitable solvent under dehydration without chemical modification. In large-scale industrial implementation N-methylmorpholine-N-oxide (NMMO) is currently used a solvent, nonetheless it is known that other solvents, such as ionic liquids, can also be used for the process. The solution is then filtered and, for the production of fibers, subsequently extruded through spinning nozzles into an air gap where they are drawn and coagulated by means of a moist airstream and then are fed into a coagulation bath containing an aqueous NMMO-solution. Subsequently the fibers can be further processed, e.g. washed, bleached, finished, crimped, cut to staple fibers, etc.

Further, processes for manufacturing of cellulosic products are known that can use an alkaline spin bath comprising a salt. To prepare the spinning solution, cellulose is dissolved in an aqueous alkaline medium at a controlled temperature. Such processes are herein generally denoted as "cold-alkali process".

The term "plain fiber", as it is used herein, refers to regenerated cellulosic fibers within the specified titer range. Preferably, the plain fibers are standard regenerated cellulosic fibers, such as lyocell fibers, viscose fibers or modal fibers or less common regenerated cellulosic fibers either available on the market or produced for the task. The percentage of the plain fibers in the nonwoven substrate is adjusted to the desired properties of the nonwoven substrate. As the plain fibers generally are less expensive than the stiffening fibers, a high percentage of plain fibers reduces the costs of the nonwoven substrate. A lower percentage of plain fibers allows for the production of higher quality nonwoven substrates and to raise the achievable parameters for these nonwoven substrates. As used herein, also a mixture of fiber types of different kind, different length and/or different titer can be considered plain fibers, as long as they all fall into the definition given in the independent Claims.

The term "stiffening fibers", as it is used herein, denotes fibers with a significantly elevated flexural rigidity in at least one bending direction when compared to the respective values for the plain fibers. This elevated rigidity is achieved by providing the fibers with a stiffening cross-section. Preferably, the stiffening cross-section can be a thickened round or naturally grown cross-section or a cross-section with a multilobal cross-section profile. As used herein the term "cross-section" refers to the form and size of the cross-section of a fiber, whereas the term "cross-section profile" refers to the form of the outline of the cross-section. As used herein, also a mixture of fiber types of different kind, different length and/or different titer can be considered stiffening fibers, as long as they all fall into the definition given in the independent Claims. Preferred examples for stiffening fibers include regenerated cellulosic fibers of any cross-section profile with a titer of 2.0 dtex or more, fibers with a multilobal cross-section profile and a titer of 1.5 dtex or more and/or natural fibers having an elevated flexural rigidity due to their cross-section profile and/or due to their titer and/or due to their microscopic cellulose structure. Natural fibers can be considered to have an elevated flexural rigidity if their flexural rigidity is in the same range as or stronger than the flexural rigidity measured for stiffening fibers comprising regenerated cellulosic fibers having a stiffening cross-section.

The term "mechanical crimp", as it is used herein, denotes a highly regular crimp pattern that has been imposed onto the fibers by mechanical means before cutting the fibers. The mechanical crimp can be produced by passing the still uncut fiber tow through a crimping device such as a stuffer box or specially profiled rollers that press the fiber tow into a wavy pattern before they are cut to staple fibers in a cutter. The resulting staple fibers have a characteristic appearance, wherein bundles of fibers can be identified that are arranged in an exactly parallel wavy pattern. Each fiber shows a regular succession of essentially straight segments of essentially identical length followed by sharp bends which produces a zig-zag pattern. The crimp of all fibers show essentially the same length and curvature which gives them a regular appearance.

As opposed to this, the term "natural crimp" designates a crimp pattern of fibers that comprises waves of different and randomly distributed curvature and length. Such fibers resemble more closely to crimp of some natural fibers, such as cotton or wool. Mechanically crimped fibers and naturally crimped fibers are per se known in the art and the person skilled in the art is able to distinguish mechanically crimped fibers from naturally crimped fibers based on the appearance and based on the production technique. Although mechanical crimping is rather common for non-cellulosic synthetic fibers, also regenerated cellulosic fibers can be mechanically crimped with essentially the same techniques.

The term "dry web-formation technique", as it is used herein, denotes a production method in which the step of assembling the fibers to form the fibrous layer is done in a dry or predominantly dry state, as opposed to wetlaid-techniques, where the fibers are suspended in a liquid medium. Preferred examples of dry web-formation techniques are the well-known dry-laid and air-laid techniques.

According to a preferred embodiment of the nonwoven substrate according to the present disclosure, a mechanical crimp of the plain fibers and/or the stiffening fibers can have a crimp frequency of between 15 and 50 crimps per 10 cm, preferably between 20 and 45 crimps per 10cm and/or a natural and/or mechanical crimp of the plain fibers and/or the stiffening fibers can have a crimp percentage of more than 5%, preferably more than 7%.

The term "crimp frequency", as it is used herein, denotes the mean number of crimps (i.e. mechanically induced bends) in a given length of fiber. One characteristic of mechanically crimped fibers is the high regularity of the crimping pattern, wherein each fiber shows a very regular succession of rather straight segments followed by a sharp bend. Compared to naturally crimped fibers, the fiber frequency of mechanically crimped fibers can be easily assed by counting the bends on a given length of fiber.

The crimp frequency of a fiber can be assessed according to the following protocol:

a. A fiber tow portion of 1 meter length is laid on a bench in order to obtain the strands for crimp level checks.

b. Twelve strands of fiber with a length between 12 and 15cm are cut from the fiber tow portion. The strands shall be cut randomly from across the full width of the tow band but not from the very edges of the tow.

c. Each strand is clipped onto a clipping board with a black background, the bottom of which measures 10 cm from the clip.

d. The strand is then extended in order to pull out all the crimp, ensuring that the strand is not pulled out of the clip. If it is not possible to pull out all the crimp from the strand then it is probably too large. In this case the sample shall be discarded and a new strand from the sample shall be produced.

e. With the sample extended, the tow is marked at the 10cm mark.

f. The sample is then allowed to relax and the number of crimps over this 10cm length, between the clip and the mark previously made, is counted.

g. Steps c. to f. are repeated for all twelve stands and the average number of crimps for all twelve strands is calculated as the crimp frequency.

The term "crimp percentage", as it is used herein, is defined as the difference between the lengths of the straightened fiber and the length of the crimped fiber (expressed as a percentage of the straightened length).

The crimp percentage can be determined according to the following protocol:

a. From a conditioned fiber sample 12 strands of fibers are carefully taken with a pincer.

b. From each strand a single fiber is carefully extracted with a pincer without stretching the fiber.

c. The fibers are loaded into a crimp tester, such as Vibrotex 400 available from Rycobel group, Belgium, according to the specifications of the respective manufacturer.

d. The fibers are loaded into the crimp tester's clamps at a minimum pretension of 1.5 mg/dtex). The clamp length is chosen as long as possible according to the fiber length and the crimp tester capacities.

e. The crimp is carefully removed by extending the fiber up to a tension level which allows to extrapolate to the standard crimp removal tension of 1 cN/tex.

f. The crimp percentage of the fiber is calculated as the difference between the lengths of the straightened fiber and the length of the pretensioned crimped fiber (i.e. the clamp length) expressed as a percentage of the straightened length.

g. Steps c. to f. are repeated for all twelve stands and the average number for all twelve strands is calculated as the crimp percentage.

According to the present disclosure, a fiber can in any case be considered having a natural crimp if it has a crimp frequency of at least 5%.

According to another preferred embodiment of the nonwoven substrate according to the present disclosure, the stiffening cross-section can comprise a round or flat or hollow or multilobal or trilobal profile.

Cellulosic man-made fibers with a round cross-section are available e.g. by the the lyocell process, while the viscose process generally produces non-round fibers with a rippled surface, e.g. cloud-shaped or resembling to a celery stick. Modal-fibers mostly have a kidney-shaped cross-section. Special production techniques also allow for the production of regenerated cellulosic fibers with a flat or multilobal profile, e.g. according to the viscose or lyocell process.

In the context of the present disclosure, the term "round" means that the maximum ovality, i.e. the difference between biggest and smallest outer diameter of the fibers is 20% or less, preferably 10% or less. Round fibers are preferable for their easy production.

The term "multilobal", as it is used herein, denotes a profile comprising three or more distinct protrusions, also called "lobes". As compared to a notional circular profile of identical area having the same centroid as the profile at hand, a distinct protrusion can be seen in any form that extends between two points of minimal distance to the centroid and has a maximal distance to the centroid of at least 20% more than the larger one of these the points of minimal distance. If the profile comprises at least three such forms, the profile is called multilobal. A trilobal profile is a multilobal profile with three distinct protrusions. As compared to round fibers, multilobal fibers are more complicated and expensive to produce, but show a higher stiffness at same titer.

A flat profile is a profile that is neither round nor multilobal and comprises a maximal outer diameter (i.e. length) and a minimal outer diamter (i.e. width), wherein the maximal outer diameter measures at least twice the length of the minimal outer diameter. Compared to round fibers, flat fibers show a good stiffness at reduced titer, but are more easily produced than multilobal fibers.

The term "hollow fiber", as it is used herein, denotes a fiber with a cross-section profile that comprises a lumen which is encircled by the fiber structure. One practical example for hollow fibers are the fibers being produced and offered by Kehlheim Fibers under the trademark Bramante(TM).

According to another preferred embodiment of the nonwoven substrate according to the present disclosure, the stiffening cross-section can be selected from a flat profile at a titer ranging from 2.0 to 4.0 dtex, a round profile at a titer ranging from 2.5 to 7.0 dtex or a multilobal, preferably trilobal, profile at a titer ranging from 1.5 to 5.0 dtex.

These ranges allow for an optimal use of the specific properties of fibers with different profiles.

According to another preferred embodiment, the nonwoven substrate according to the present disclosure can have a basis weight of between 30 and 85 g/m$^2$, preferably 35-70 g/m$^2$.

The nonwoven substrate must be thick enough to fulfill its assigned tasks, but for commercial, economic and environmental reasons should be as thin as possible. The ranges for the basis weight as disclosed herein allow to fit the properties of the nonwoven substrate to many different functions and tasks. So, the nonwoven substrate can be adjusted to a big range of different products. The basis weight can preferably be measured according to the Harmonized Nonwoven Standard Procedure NSWP 130.1.R0(15) [EN].

In another preferred embodiment of the nonwoven substrate according to the present disclosure, the plain fibers can have a mean cut length of between 28 mm and 55 mm and/or the stiffening fibers can have a mean cut length of between 30 mm and 70 mm.

These lengths are preferable for dry-web-formation techniques and provide for a good result in the light of the present disclosure. Further, at least for a part of the fibers in the nonwoven substrate, standard available fibers can be used.

According to another preferred embodiment of the nonwoven substrate according to the present disclosure, the stiffening fibers can have a mean cut length that is at least 25% longer than the mean cut length of the plain fibers.

This improves the structural stability of the nonwoven substrate. The effect can be increased if during production of the nonwoven substrate at least one fiber fleece that is used to produce the nonwoven substrate is condensed in a carding machine. This also increases the tensile strength of the nonwoven substrate, especially in cross direction.

In still another preferred embodiment of the nonwoven substrate according to the present disclosure, the combined weight of the plain fibers and the stiffening fibers can account for 90 % to 100 % of the weight of the fibrous layer, preferably for 98% to 100 % of the weight of the fibrous layer.

This allows for a simple composition of the nonwoven substrate. E.g. the nonwoven substrate can be composed of (or essentially composed of) only two different kinds of fibers, namely plain fibers and stiffening fibers. The plain fibers can be all of the same kind or they can comprise a blend of different pain fibers according to the definitions disclosed herein. In the same way, the stiffening fibers can be all of the same kind or they can comprise a blend of different stiffening fibers according to the definitions disclosed herein.

According to another embodiment, the nonwoven substrate according to the present disclosure can have a wet tensile strength in cross direction and/or machine direction of between 8 and 50 N/5 cm.

The wet tensile strength can preferably be measured according to the Harmonized Nonwoven Standard Procedure NWSP 110.4.R0 (15) [EN]. The wet tensile strength can be adjusted to the needs and to the parameters disclosed herein by selectively adjusting the production parameters and the composition of the fiber feedstock that is used to produce the nonwoven substrate according to the teachings disclosed herein.

The nonwoven substrate according to the present disclosure can exhibit a thickness reduction when wetted of less than 20%.

The thickness reduction when wetted (herein also called "wet collapse") can be determined according to the formula

$$\text{wet collapse [\%]} = (1 - (\text{wet thickness} / \text{dry thickness}))*100$$

The dry thickness can be measured according to he Harmonized Nonwoven Standard Procedure NWSP 120.6.R0 (15) [EN]. For measuring the wet thickness, the following protocoll is used:

- 30 Samples, each with a size of 10x10 cm, are cut out of the nonwoven substrate to be tested

- the 30 samples are stacked over each other and the whole stack is weighed to define the sample weight of whole staple before water was applied

- the whole staple of 30 samples is dipped into water until fully soaked with water for 1 min

- the wet staple of 30 samples is gently withdrawn from water and placed onto a grid so that water can drip off. This is done under norm climate conditions (i.e. 23°C and 50% relative humidity)

- the stack of 30 staples is weighed twice a day until the water content of the fabric samples (i.e. the weight) is in the range of 2,5-3,5 times the dry basis weight

- when the stack of 30 samples is in the defined water content range the uppermost 10 samples (i.e. Samples 1-10) are withdrawn and discarded

- Samples 21-30 are also discarded

- Samples 11-20 from the middle of stack are used to test wet fabric thickness

- For testing the wet fabric thickness the same protocol (according to according to NWSP 120.6.R0 (15) [EN]) as for the measurement of the dry thickness, but instead of measuring conditioned samples, the wet fabric samples are used.

A low wet collapse is especially important for nonwovens that are provided for taking up liquid or fluid substances, such as water, detergent, lotion or the like. For example, wet wipes are wetted during packing and shall not significantly reduce their thickness during storage before use. The thickness reduction when wetted ("wet collapse") can be adjusted to the needs and to the parameters disclosed herein by selectively adjusting the production parameters and the composition of the fiber feedstock that is used to produce the nonwoven substrate according to the teachings disclosed herein.

According to one preferred embodiment, the nonwoven substrate can have a wet elongation of 25-70 % / 5 cm in machine direction (MD) and 35-110 % / 5 cm in cross direction (CD).

A wet elongation in this range is especially preferable for wet wipes. According to the teachings disclosed herein, the wet elongation can be adjusted to the needs by the production parameters and the composition of the fiber feedstock that is used to produce the nonwoven substrate. The wet elongation can be measured according to the Harmonized Nonwoven Standard Procedure NWSP 110.4.

The present disclosure further relates to an industrial or consumer product comprising a nonwoven substrate according to any embodiments disclosed herein, wherein the product is preferably selected from a list comprising wipes, wet wipes, fluid management layers, such as an acquisition and distribution layer (ADL).

In such products the special properties of the nonwoven substrates according to the present disclosure, such as the low wet collapse, can beneficially be exploited.

Further, the present disclosure relates to a method for producing a fibrous layer, the method comprising the steps of

a. providing feedstock fibers comprising from 10 % w/w to 90 % w/w plain fibers, preferably from 20% w/w to 80% w/w plain fibers, and from 10 % w/w to 90 % w/w stiffening fibers, preferably from 20% w/w to 80 % w/w stiffening fibers, wherein the plain fibers are regenerated cellulosic fibers with a titer of between 1.2 and 1.9 dtex and the stiffening fibers are regenerated or natural cellulosic fibers with a stiffening cross-section,

b. opening and mixing the feedstock fibers to produce a homogeneous fiber fleece,

c. carding the fiber fleece to form the fibrous layer,

wherein at least one of the plain fibers and/or the stiffening fibers are provided with a mechanical or natural crimp.

The method allows for the production of a fibrous layer as an intermediate product from which a plurality of advantageous products can be made, for example nonwoven substrates with a high wetting stability and a low wet collapse.

According to a preferred embodiment or the method disclosed herein, carding the fiber fleece can comprise condensing the fiber fleece.

Condensing is a production step that is generally done by a condenser arranged at the exit of a carding machine after the doffer. The condenser usually comprises at least one condensing roll combing the upstream roll (i.e. the doffer or an upstream condensing roll) with a decreasing surface speed gradient. The fibrous layer that is transported by the upstream roll at a relatively higher speed gets condensed when it is passed to the condensing roll working at a relatively lower speed. By condensing the fiber fleece the fibers in the fleece get rearranged in a three-dimensional way. This renders the fiber fleece more bulky and further increases the stability (especially in cross direction) and the wet stability of the fiber fleece or the nonwoven substrate produced therefrom, respectively.

The condensing rate (i.e. the ratio of the entry-speed of the fiber-fleece into the condenser and the exit-speed of the fiber fleece leaving the condenser) shall be adjusted to the desired basis weight of the fibrous layer. Nonetheless, the condensing rate cannot be selected too high, as this could overload the card wires with fibrous material which could lead to production losses or even machine damages.

In connection with the teachings disclosed herein, an overall condensing rate ranging from 1.2 to 8 is believed to fulfill most needs, wherein optimized results are believed to be found at a condensing rate ranging from 1.7 to 5. Also the number of condensing rolls shall be taken into account, as a higher number of condensing rolls generally allows a higher condensing rate. For each condensing roll a condensing rate ranging from 1.2 to 4 is believed to fulfill most needs, wherein optimized results are believed to be found at a condensing rate ranging from 1.7 to 3.

According to another preferred embodiment of the method disclosed herein, the method for producing a nonwoven substrate can comprise the steps of providing a layer feedstock comprising at least one fibrous layer produced according to any of the methods disclosed herein and subjecting the at least one layer to a web-bonding treatment.

The web-bonding can be done by any reasonable method known in the art, such as needle-punching or spunlacing / hydroentanglement. Depending on the required thickness of the nonwoven substrate and the thickness of the fibrous layer or layers, the feedstock can comprise multiple fibrous layers (of the same or different kind) that are stacked and commonly subjected to the web-bonding step. Further, different production techniques, such as crosslapping of two or more fibrous layers can be used to produce the nonwoven substrate.

In another preferred embodiment, a nonwoven substrate according the present disclosure can be used for the production of an industrial or consumer good, perferably selected from a list comprising wipes, wet wipes, fluid management layers, such as an acquisition and distribution layer (ADL).

If not specifically stated otherwise, all measurements disclosed herein are performed on samples that have been conditioned according to NWSP 003.R0 (15) [EN] to norm climate conditions of 23°C (+/- 2.0 °C) and 50% humidity (+/- 4.0 %).

**Brief Description of the Drawings**

Hereinafter, exemplary embodiments of the invention are described with reference to the drawings, wherein

Fig. 1 shows a schematic diagram of exemplary process steps for producing a nonwoven substrate according to the present disclosure,

Fig. 2 shows a diagram of visualising properties of different nonwoven substrate samples and

Fig. 3 shows another diagram of visualising properties of different nonwoven substrate samples

**Detailed Description of the Drawings**

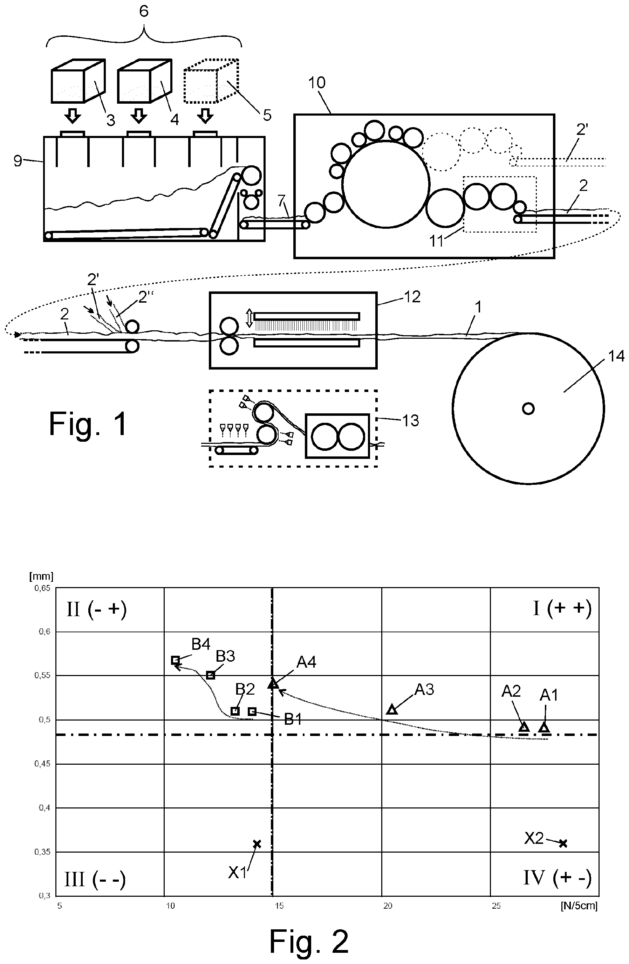

Fig. 1 shows a diagrammatic representation of production steps in a process that can be used for manufacturing a nonwoven substrate 1 according to the present disclosure.

As a starting material, feedstock fibers 6 are provided to an opener 9, where the different feedstock fibers 6 are opened and thoroughly mixed. The feedstock fibers 6 can comprise different types of fibers in different mix ratios according to the examples and definitions within the present disclosure and the Claims. Generally, the feedstock fibers 6 comprise plain fibers 3 and stiffening fibers 4. The plain fibers 3 and the stiffening fibers 4 are both regenerated cellulosic fibers. Additionally the feedstock fibers 6 can comprise additional fibers 5 of any desired kind or properties, as long as the use of these additional fibers 5 is technically feasible and useful.

As a general target, the feedstock fibers 6 comprise from 10 % w/w to 90 % w/w plain fibers 3, preferably from 20% w/w to 80% w/w plain fibers 3, and from 10 % w/w to 90 % w/w stiffening fibers 4, preferably from 20% w/w to 80 % w/w stiffening fibers 4.

The plain fibers 3 can be commercially available "standard" fibers as they are commonly used for the production of nonwoven fabrics having a titer of between 1.2 and 1.9 dtex. The use of commercially available fibers reduces the cost for the production of the nonwoven substrate 1. A titer of less than 1.2 dtex is generally avoided as this would lead to higher costs and technical problems during the carding process.

The stiffening fibers 4 are characterized by a special stiffening cross-section which increases the flexural rigidity of the fiber in at least one bending direction. A stiffening cross-section can either be characterized by having a standard cross-section profile at an increased titer, such as so-called high-titer fibers, or by having an alternative cross-section profile, such as a multilobal or flat or hollow profile.

Either the plain fibers 3 or the stiffening fibers 4 or both, the stiffening fibers 4 and the plain fibers 3, are provided with a mechanical or natural crimp. Fibers having a mechanical crimp are per se known in the art. Mechanical crimping is done during fiber production before cutting while the fibers are conveyed in an uncut fiber tow. The process of mechanical crimping is very common in the field of synthetic man-made fibers, such as polyester fibers or the like. As these fibers are initially straight, the crimp is mechanically impressed on the fibers, either by use of profiled rollers or a stuffer box. Cellulosic regenerated fibers mostly are not provided with a mechanical crimp as there are rather simple processing steps which can provide them with an irregular crimp that gives the fiber a more "natural" appearance, which is generally preferred by the consumers. Generally, for applying a natural crimp, the fiber tow is cut in a wet state and collected in a fleece. By applying pressure to the still undried fleece, a natural crimp is imposed on the fibers. This leads to a crimp pattern that closely resembles to the crimp pattern seen in natural fibers such as wool or cotton. Cellulosic fibers can also be provided with a mechanical crimp and there are cellulosic fibers available that are mechanically crimped, especially in the field of lyocell fibers. A technician skilled in the field can easily discern mechanically crimped fibers from naturally crimped fibers based on their crimp pattern and their appearance. For example, a crimp frequency can easily be determined with mechanically crimped fibers, whereas such a crimp frequency is not defined (and cannot be determined) for naturally crimped fibers. A crimp percentage can be measured for all crimped fibers and the definition of crimp percentage can be applied to mechanically crimped fibers and naturally crimped fibers.

The additional fibers 5 can be selected according to the needs of the producer, e.g. to give special properties to the nonwoven substrate 1 to be produced. Preferably, the additional fibers are present in an proportion of between 0 and 20 % of the weight of the feedstock fibers 6. In a preferred embodiment, only a very low proportion of additional fibers 5 (e.g. only 2 % per weight) or no additional fibers at all are present in the feedstock fibers 6. I.e. the feedstock fibers 6 (and therefore the nonwoven substrate 1 to be produced) consists of 100% (or almost 100%) plain fibers 3 and stiffening fibers 4

In the opener 9 the feedstock fibers 6 are opened. Then the fibers are thoroughly mixed, which can be done by a blending machine or a continuous fiber blender, and then transported to a card chute which produces a homogenous fiber fleece 7 that is then fed into a carding machine 10 for carding the fiber fleece 7. The homogeneous fiber fleece 7 comprises the plain fibers 3, the stiffening fibers 4 and optionally the additional fibers 5 in the same composition as provided by the feedstock fibers 6, but homogeneously mixed.

Carding ensures that each of the different fibers contribute to the desired properties of the nonwoven substrate 1 and that the full potential of the raw material is exploited. The carding machine 10 can optionally be provided with a condenser 11 in which the thickness (or basis weight) of the fiber fleece 7 is increased by the use of at least one condensing roll that reduces the transport speed of the fiber fleece 7 so that it gets "pushed" together in its direction of transport. By this condensing step the resulting fibrous layer 2 that exits the carding machine gets thicker and the fibers therein get arranged in a more three-dimensional way. This increases the stability and the strength of the fibrous layer 2.

Carding machines are per se known in the art and many different techniques and equipment for carding exist. For example, high-performance carding machines can be used that comprise more than one fiber take-off sections (such as a doffer roll), so that two or more fibrous layers 2, 2' can be produced by one carding machine. This is indicated in Fib. 1 in doted lines. After each fiber take-off section, a condenser 11 can be arranged. Also, two or more carding machines, which can be similar or identical to each other, can be used in a parallel manner. For example, two carding machines, each producing two fibrous layers 2, 2', can be used in a parallel arrangement, so that four fibrous layers are simultaneously produced. The simultaneously produced fibrous layers can then be combined in a parallel manner or crosswise (which is reffered to as crosslapping) and then be further processed.

After carding the fibrous layer 2 can be stacked with other fibrous layers 2', 2" of the same kind or of a different kind to increase the thickness of the product to a desired quality. In Fib. 1 two additional fibrous layers 2', 2" that are added to the first fibrous layer 2 are schematically and exemplarily depicted. The fibrous layer(s) 2, 2', 2" are then bonded in a bonding machine 12. The fibrous layers can be stacked in a parallel way or crosswise according to a crosslapping-technique which can improve the properties of the nonwoven substrate to be produced. The bonding machine can use any technique known in the art such as hydroentanglement (also called spunlacing) or needle-punching. In Fig. 1 a needle-punching machine is schematically depicted in the production line. A hydroentanglement unit 13 (or spunlacing machine) is schematically and exemplarily depicted below as an alternative processing device. Also, several different bonding techniques can be used in combination, if this is technically feasible and commercially advantageous.

After bonding, the finished nonwoven substrate 1 can be stored or further processed. Generally, the nonwoven substrate 1 is packed in rolls by a winding machine 14 and transported to a store or directly to a customer.

As commonly understood, the terms "fiber fleece", "fibrous layer" and "nonwoven substrate" be used in a synonymous way. To improve the readability and the understanding of the present disclosure, the term "homogeneous fiber fleece 7" is herein used for the fleece after opening/mixing before carding. The term "fibrous layer 2" is herein used for the carded intermediate product and the term "nonwoven substrate 1" is herein used for the fleece after the bonding step. It has to be pointed out that this distinction is intended for the ease of understanding only and shall not be construed to reduce the scope of the present disclosure or claims over their intended meaning.

As will be further shown in the examples below, it has been surprisingly found that nonwoven substrates made according to the teachings herein have special properties that can make the production of high-quality nonwoven substrates easier and cheaper.

### Examples:

Several nonwoven substrates 1 have been produced according to the techniques described herein using a different feedstock fiber composition. The feedstock fibers were opened, homogenuosly mixed to form a homogeneous fiber fleece which was then fed to a carding machine (Trützschler Roller Card TWF-NC) comprising two take-off sections, each being provided with a condenser with two condensing rolls.

The condensing rate for each nonwoven substrate sample is given in the form of two values: the condensing rate for the first condensing roll and the condensing rate for the second condensing roll. Each condensing rate is defined as the surface speed of the preceding roll divided by the surface speed of the respective condensing roll. For example, a condensing rate of 1.8/1.3 means that the speed of the first condensing roll was set to a value of the speed of the preceding roll (i.e. the doffer roll) divided by 1.8 and the speed of the second condensing roll was set to a value of the speed of the first condensing roll divided by 1.3. In this example, if the doffer roll would work at a speed of 100m/min, the speed of the first condensing roll would be 100/1.8=55.56 m/min and the speed of the second condensing roll would be 55.6/1.3=42.74 m/min.

By condensing the fibers in the fibrous layer 2 get "restructured" in a three-dimensional way which leads to a higher bulkiness and tensile strength, especially in cross direction.

The condensing rates have to be selected in a reasonable range, as the card wires of the rolls can get overloaded at high condensing rates which could lead to production losses or even damage the equipment. Also, the condensing rates have to be selected according to the desired basis weight of the nonwoven substrate to be produced. For example, if the target basis weight is 45 g/m$^2$ after bonding and conditioning, this basis weight has to be divided by two to account for the two take-off sections and, according to practical experience, this theoretical value has to be somewhat reduced to compensate for the following bonding and conditioning steps. For practical reasons, in the configuration that was used for producing the samples, a basis weight of 21.3 g/m$^2$ for each carded and condensed fibrous layer was set. Two fibrous layers were stacked in a parallel way, bonded and conditioned to give the nonwoven substrate with the target basis weight.

The bonding was performed by spunlacing on a spunlacing facility "Trützschler Mini Jet" having 5 water jet beams.

Two different production settings were used and the settings were adjusted to give a nonwoven substrate with a nominal basis weight of 45 g/m$^2$.

- Setting One:

  ◦ condensing rate 2,2/1,7

◦ line speed 50 m/min

◦ spunlace pressures: 40/30/30/65/75 bar

◦ jet strip 120µm 40 HPI single row

- Setting Two:

◦ condensing rate 2,4/1,9

◦ line speed 50 m/min

◦ spunlace pressures: 40/30/30/75/85 bar

◦ jet Strip 120µm 40 HPI single row

Four different sample groups, A, B, C and D, were produced, each comprising four different percentages for plain fibers and stiffening fibers, namely 20% stiffening fibers (A1, B1, C1, D1), 40% stiffening fibers (A2, B2, C2, D2), 60% stiffening fibers (A3,B3, C3, D3) and 80 % stiffening fibers (A4, B4, C4, D4).

In Sample Groups C and D two samples were produced for each group: one sample was produced according to Setting One and a second sample was produced according to Setting Two.

In Sample Groups A and B two different subgroups (indicated by a number extension /1 or /2, respectively) were produced and measured, each subgroup representing a slight variation of either the plain fiber (CV1.7/38 instead of CV 1.7/40 in Group A) or the stiffening fiber (CV trilobal 3.3/50 instead of CV trilobal 3.3/40 in Group B) and for each of these subgroups one sample was produced according to Setting One and a second sample was produced according to Setting Two. For the sake of completeness the measurement results in this groups are given for the subgroups (e.g. A1/1, etc.) and as an averaged result (e.g. the measurement results given for A1 correspond to the averaged results for A1/1 and A1/2, etc.)

An overview of the samples is given in Table 1 below.

Table 1 - Sample composition

| Sample No | Plain Fiber Type | PF % | Stiffening Fiber Type | SF % |
|-----------|------------------|------|-----------------------|------|
| A1/1 | CV 1.7/40 | 80 | CLY 6.7/50 | 20 |
| A1/2 | CV 1.7/38 | 80 | CLY 6.7/50 | 20 |
| A2/1 | CV 1.7/40 | 60 | CLY 6.7/50 | 40 |
| A2/2 | CV 1.7/38 | 60 | CLY 6.7/50 | 40 |
| A3/1 | CV 1.7/40 | 40 | CLY 6.7/50 | 60 |
| A3/2 | CV 1.7/38 | 40 | CLY 6.7/50 | 60 |
| A4/1 | CV 1.7/40 | 20 | CLY 6.7/50 | 80 |
| A4/2 | CV 1.7/38 | 20 | CLY 6.7/50 | 80 |
| B1/1 | CV 1.7/40 | 80 | CV trilobal 3.3/40 | 20 |
| B1/2 | CV 1.7/40 | 80 | CV trilobal 3.3/50 | 20 |
| B2/1 | CV 1.7/40 | 60 | CV trilobal 3.3/40 | 40 |
| B2/2 | CV 1.7/40 | 60 | CV trilobal 3.3/50 | 40 |
| B3/1 | CV 1.7/40 | 40 | CV trilobal 3.3/40 | 60 |
| B3/2 | CV 1.7/40 | 40 | CV trilobal 3.3/50 | 60 |
| B4/1 | CV 1.7/40 | 20 | CV trilobal 3.3/40 | 80 |
| B4/2 | CV 1.7/40 | 20 | CV trilobal 3.3/50 | 80 |
| C1 | CV 1.7/40 | 80 | CLY 3.3/60 | 20 |

(continued)

| Sample No | Plain Fiber Type | PF % | Stiffening Fiber Type | SF % |
|---|---|---|---|---|
| C2 | CV 1.7/40 | 60 | CLY 3.3/60 | 40 |
| C3 | CV 1.7/40 | 40 | CLY 3.3/60 | 60 |
| C4 | CV 1.7/40 | 20 | CLY 3.3/60 | 80 |
| D1 | CLY 1.7/51 | 80 | CV trilobal 3.3/50 | 20 |
| D2 | CLY 1.7/51 | 60 | CV trilobal 3.3/50 | 40 |
| D3 | CLY 1.7/51 | 40 | CV trilobal 3.3/50 | 60 |
| D4 | CLY 1.7/51 | 20 | CV trilobal 3.3/50 | 80 |

Two comparison samples, X1 and X2, were produced according to the same protocol, but only consisting of standard plain fibers. Comparison Sample X1 consisted of 100% standard viscose fibers (CV 1.7/40). Comparison Sample X2 consisted of a blend of 70% standard lyocell fibers (CLY 1,7/38) and 30% standard viscose fibers (CV 1.7/40).

In the following, the abbreviations for the fiber types, as used in the tables and the text, are further explained:

**CV 1.7/40**

Standard viscose staple fiber with a titer of 1.7 and a cut length of 40 mm. The fibers had a natural crimp with a crimp frequency in the range of 5 to 7 %

**CV 1.7/38**

Essentially identical to CV 1.7/40, but whit a cut length of 38 mm.

**CLY 1.7/51**

Standard lyocell staple fiber with a titer of 1.7 and a cut length of 51 mm. The fibers had a mechanical crimp with a crimp frequency of 40 crimps/10 cm (range of tolerance: 35-45 crimps/10 cm)

**CLY 6.7/50**

Lyocell staple fibers with a round stiffening profile having a titer of 6.7 and a cut length of 50 mm. The fibers had a natural crimp with a crimp frequency in the range of 5 to 7%.

**CV trilobal 3.3/50**

Viscose fibers having a trilobal cross-section, a titer of 3.3 and a cut length of 50 mm. The fibers are commercially available under the trade name "Viscostar". The fibers had a natural crimp with a crimp frequency in the range of 5 to 7 %.

**CV trilobal 3.3/40**

Essentially identical to CV trilobal 3.3/50, but with a cut length of 40 mm.

**CLY 3.3/60**

Lyocell fibers having a round stiffening cross-section with a titer of 3.3 and a cut length of 60 mm. The fibers were mechanically crimped with a crimp frequency of 30 crimps/10 cm (range of tolerance: 22-35 crimps/10 cm).

**CLY 1.7/38**

Standard lyocell staple fiber with a titer of 1.7 and a cut length of 38 mm. The fibers had a natural crimp with a crimp frequency in the range of 5 to 7 %.

For all Samples the fabric thickness in mm was measured in dry and in wet state according to the protocol described herein. The wet collapse was calculated based on these measurements Further, the Wet tensile strength in Cross direction (CD) was measured.

Results of the measurements are given in Table 2 below.

Table 2 - Measurement results

| Sample | Basis Weight [g/m²] | Wet CD tensile strength [N/5cm] | Wet fabric thickness [mm] | Dry fabric thickness [mm] | Wet Collapse [%] |
|---|---|---|---|---|---|
| A1/1 | 44,20 | 38,55 | 0,49 | 0,48 | -1,04 |
| A2/1 | 44,45 | 37,45 | 0,51 | 0,50 | -1,00 |
| A3/1 | 41,55 | 28,15 | 0,53 | 0,53 | 0,00 |

(continued)

| Sample | Basis Weight [g/m²] | Wet CD tensile strength [N/5cm] | Wet fabric thickness [mm] | Dry fabric thickness [mm] | Wet Collapse [%] |
|---|---|---|---|---|---|
| A4/1 | 44,10 | 21,75 | 0,54 | 0,59 | 7,69 |
| A1/2 | 46,10 | 16,20 | 0,49 | 0,54 | 9,26 |
| A2/2 | 44,80 | 16,15 | 0,48 | 0,53 | 10,38 |
| A3/2 | 46,45 | 13,25 | 0,49 | 0,55 | 10,09 |
| A4/2 | 43,30 | 8,40 | 0,53 | 0,61 | 12,40 |
| B1/1 | 44,00 | 14,55 | 0,50 | 0,52 | 3,85 |
| B2/1 | 45,00 | 13,35 | 0,53 | 0,55 | 3,67 |
| B3/1 | 42,50 | 12,70 | 0,57 | 0,61 | 7,38 |
| B4/1 | 44,50 | 10,30 | 0,56 | 0,68 | 17,78 |
| B1/2 | 45,50 | 13,85 | 0,53 | 0,55 | 3,67 |
| B2/2 | 44,50 | 13,30 | 0,52 | 0,57 | 9,65 |
| B3/2 | 45,00 | 11,80 | 0,54 | 0,61 | 11,57 |
| B4/2 | 46,50 | 10,60 | 0,59 | 0,66 | 10,69 |
| A1 | 45,15 | 27,38 | 0,49 | 0,51 | 4,41 |
| A2 | 44,63 | 26,80 | 0,49 | 0,52 | 4,85 |
| A3 | 44,00 | 20,70 | 0,51 | 0,54 | 5,14 |
| A4 | 43,70 | 15,08 | 0,54 | 0,60 | 10,08 |
| B1 | 44,75 | 14,20 | 0,51 | 0,53 | 3,76 |
| B2 | 44,75 | 13,33 | 0,52 | 0,56 | 6,73 |
| B3 | 43,75 | 12,25 | 0,55 | 0,61 | 9,47 |
| B4 | 45,50 | 10,45 | 0,57 | 0,67 | 14,29 |
| C1 | 44,35 | 18,40 | 0,49 | 0,53 | 7,55 |
| C2 | 46,45 | 19,90 | 0,51 | 0,53 | 3,77 |
| C3 | 47,95 | 22,90 | 0,53 | 0,57 | 7,02 |
| C4 | 49,55 | 27,35 | 0,56 | 0,62 | 9,68 |
| D1 | 45,95 | 26,45 | 0,56 | 0,57 | 1,75 |
| D2 | 49,00 | 20,30 | 0,58 | 0,61 | 4,92 |
| D3 | 48,10 | 16,70 | 0,61 | 0,64 | 4,69 |
| D4 | 47,25 | 13,35 | 0,63 | 0,71 | 11,27 |
| X1 (comp.) | 44,85 | 14,28 | 0,36 | 0,45 | 20,00 |
| X2 (comp.) | 44,45 | 28,50 | 0,36 | 0,47 | 23,40 |

The wet collapse of the example samples are all in a range from between 1 % and 15%, whereas the comparison samples have a wet collapse of 20% or more. Interestingly the wet collapse generally tends to depend on the ratio of plain fibers and stiffening fibers, wherein a rather low amount of stiffening fibers leads to a lower wet collapse. This suggests that the wet collapse can be adjusted to the needs by a proper selection of this fiber ratio.

It should be noted that for practical purposes the absolute value of the wet thickness of the nonwoven substrate is more important than the percental wet collapse. As can be seen from Table 2, already the dry thickness shows considerable variations, although essentially the same procedures have been used for the production of the nonwoven substrates of the samples. Also, all nonwoven substrates of the samples have been produced with the same basis weight settings, i.e. the calculated basis weight has been set to 45 $g/m^2$. Practically, the measurement results for the basis weights slightly vary in value, but all measurements are within the general tolerance for nonwoven fabrics of +/- 10%. Therefore all example and comparison samples can be considered to have essentially the same basis weight.

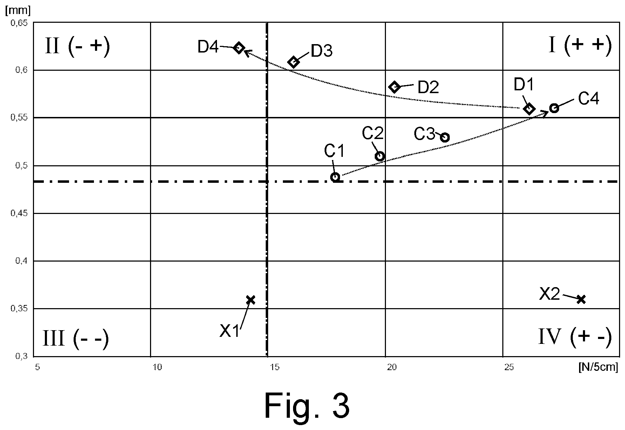

The values for the wet thickness and the wet tensile strength are graphically depicted in the diagrams shown in Fig. 2 and Fig. 3.

The charts in Fig. 2 and Fig. 3 compare the wet tensile strength (CD) in N/5cm (axis of abscissas) with the wet fabric thickness (axis of ordinates). The chart is grouped by two guidance values (dotdashed lines) into for quadrants I, II, III and IV.

The horizontal guidance value was delineated at a wet fabric thickness of 0.48 mm. Fabrics that exceed this value (quadrants I and II) can be considered to have a very good wet thickness (and therefore a good wet collapse) for most uses and also meet many quality requirements demanded by customers. Also nonwoven substrates below this guidance value (quadrants III and IV) can be useful for some applications, but only under certain restrictions.

The vertical guidance value was delineated at a wet tensile strength (CD) of 15 N/5cm. Fabrics that exceed this value (quadrants I and IV) can be considered to have a very good tensile strength when wet and meet most quality requirements demanded by customers. Also nonwoven substrates below this guidance value (quadrants II and III) can be useful for some applications, but only under certain restrictions.

In general terms it can be noted that it is a goal for the development of new nonwoven substrates to avoid the region of quadrant III and possibly get as far as possible into the area of quadrant I, which combines a good tensile strength with a good wet thickness (and therefore a preferred low wet collapse).

It can be seen, that comparison sample X1 has only a very poor performance both, in terms of wet thickness and tensile strength. Comparison sample X2 shows a remarkably increased tensile strength, but also has a very poor performance in terms of wet thickness/wet collapse. The results for the comparison samples are consistent with the experience in this field that nonwoven substrates produced from staple fibers generally do not preserve their thickness and collapse when wet.

The results for Samples A1 to A4 (Fig. 2) shows that the wet thickness can be improved by blending stiffening fibers to the plain fibers. It should be noted that the wet thickness increases with a higher content of stiffening fibers (from A1, 20% to A4, 80%), but at the cost of a decreasing tensile strength.

An even better result for the wet thickness could be achieved by using stiffening fibers with a trilobal stiffening cross-section profile as can be seen in Samples B1-B4 (Fig. 2). Although the results for the wet thickness of the nonwoven substrates B1 to B4 are very good, the properties of the tensile strength were not in the optimal range and still gave room for improvement.

Samples C1 to C4 (Fig. 3) were produced using essentially the same plain fibers as in Samples A1-A4 and B1-B4, but this time blended with Lyocell fibers having a round stiffening cross section (titer of 3.3) and a rather high cut length of 60 mm. With these Samples all measurement results were found to be in the preferred quadrant I. Further, an increase in the amount of stiffening fibers increases both the wet thickness and the tensile strength.

Even higher values for the wet thickness could be found with Samples D1 to D4 (Fig. 3), which used standard lyocell plain fibers with a titer of 1.7 and a cut length of 51 mm and trilobal viscose fibers of nearly the same length (50 mm) and a titer of 3.3. Nonetheless, the tensile strength decreased with a higher amount of stiffening fibers in this sample group.

Given these experimental results, the current applicants draw the conclusion that it is possible to produce high quality nonwoven substrates having a low wet collapse by using a mixture of only two different fiber types. The person skilled in the art, that is aware of the teachings disclosed herein, is able to choose suitable feedstock fibers and optimize their composition and percentages to optimize the properties of the nonwoven substrate to be produced.

**Reference numbers:**

    nonwoven substrate 1
    fibrous layer 2
    plain fibers 3
    stiffening fibers 4
    additional fibers 5
    feedstock fibers 6
    homogeneous fiber fleece 7

layer feedstock 8
opener 9
carding machine 10
condenser 11
bonding machine 12
hydroentanglement unit 13
winding machine 14

**Claims**

1.  Nonwoven substrate comprising at least one fibrous layer formed by a dry web-formation technique, the fibrous layer comprising from 10 % w/w to 90 % w/w plain fibers, preferably from 20% w/w to 80% w/w plain fibers, and from 10 % w/w to 90 % w/w stiffening fibers, preferably from 20% w/w to 80 % w/w stiffening fibers, wherein the plain fibers are regenerated cellulosic fibers with a titer of between 1.2 and 1.9 dtex and the stiffening fibers are natural or regenerated cellulosic fibers with a stiffening cross-section, wherein at least one of the plain fibers and/or the stiffening fibers being provided with a mechanical crimp or a natural crimp.

2.  Nonwoven substrate according to Claim 1, wherein a mechanical crimp of the plain fibers and/or the stiffening fibers has a crimp frequency of between 15 and 50 crimps per 10 cm, preferably between 20 and 45 crimps per 10cm and/or wherein a natural and/or mechanical crimp of the plain fibers and/or the stiffening fibers has a crimp percentage of more than 5%, preferably more than 7%.

3.  Nonwoven substrate according to Claim 1 or 2, wherein the stiffening cross-section comprises a round or flat or hollow or multilobal or trilobal profile.

4.  Nonwoven substrate according to any of the Claims 1 to 3, wherein the stiffening cross-section is selected from a flat profile at a titer ranging from 2.0 to 4.0 dtex, a round profile at a titer ranging from 2.5 to 7.0 dtex or a multilobal, preferably trilobal, profile at a titer ranging from 1.5 to 5.0 dtex.

5.  Nonwoven substrate according to any of the Claims 1 to 4, wherein the nonwoven substrate has a basis weight of between 30 and 85 g/m$^2$, preferably 35-70 g/m$^2$.

6.  Nonwoven substrate according to any of the Claims 1 to 5, wherein the plain fibers have a mean cut length of between 28 mm and 55 mm and/or the stiffening fibers have a mean cut length of between 30 mm and 70 mm.

7.  Nonwoven substrate according to any of the Claims 1 to 6, wherein the stiffening fibers have a mean cut length that is at least 25% longer than the mean cut length of the plain fibers.

8.  Nonwoven substrate according to any of the Claims 1 to 7, wherein the combined weight of the plain fibers and the stiffening fibers accounts for 90 % to 100 % of the weight of the fibrous layer, preferably for 98% to 100 % of the weight of the fibrous layer.

9.  Nonwoven substrate according to any of the Claims 1 to 9, wherein the nonwoven substrate has a wet tensile strength in cross direction and/or machine direction of between 8 and 50 N/5 cm.

10. Nonwoven substrate according to any of the Claims 1 to 9, wherein the nonwoven substrate exhibits a thickness reduction when wetted of less than 20%.

11. Nonwoven substrate according to any of the Claims 1 to 10, wherein the nonwoven substrate has a wet elongation of 25-70 % / 5 cm in machine direction (MD) and 35-110 % / 5 cm in cross direction (CD).

12. Industrial or consumer product comprising a nonwoven substrate according to any of the Claims 1 to 11, wherein the product is preferably selected from a list comprising wipes, wet wipes, fluid management layers, such as an acquisition and distribution layer (ADL).

13. Method for producing a fibrous layer, the method comprising the steps of

d. providing feedstock fibers comprising from 10 % w/w to 90 % w/w plain fibers, preferably from 20% w/w to 80% w/w plain fibers, and from 10 % w/w to 90 % w/w stiffening fibers, preferably from 20% w/w to 80 % w/w stiffening fibers, wherein the plain fibers are regenerated cellulosic fibers with a titer of between 1.2 and 1.9 dtex and the stiffening fibers are regenerated or natural cellulosic fibers with a stiffening cross-section,

e. opening and mixing the feedstock fibers to produce a homogeneous fiber fleece,

f. carding the fiber fleece to form the fibrous layer,

wherein at least one of the plain fibers and/or the stiffening fibers are provided with a mechanical or natural crimp.

14. Method according to Claim 13, wherein carding comprises condensing the fiber fleece.

15. Method for producing a nonwoven substrate comprising the steps of providing a layer feedstock comprising at least one fibrous layer produced according to any of the methods of Claims 13 or 14 and subjecting the at least one layer to a web-bonding treatment.

16. Use of a nonwoven substrate according to any of the Claims 1 to 11, for the production of an industrial or consumer good, perferably selected from a list comprising wipes, wet wipes, fluid management layers, such as an acquisition and distribution layer (ADL).

Fig. 1

Fig. 2

Fig. 3

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2021/197927 A1 (CHEMIEFASER LENZING AG [AT]) 7 October 2021 (2021-10-07) * table 1 * ----- | 1-16 | INV. D04H1/425 D04H1/4258 D04H1/4391 D04H1/74 |
| A | WO 2020/254931 A1 (GRASIM INDUSTRIES LTD [IN]) 24 December 2020 (2020-12-24) * claims 1, 4, 6, 22 * ----- | 1-16 | ADD. A61F13/537 A61K8/02 |

TECHNICAL FIELDS
SEARCHED (IPC)

D04H
A61F
A61K

The present search report has been drawn up for all claims

| Place of search | Date of completion of the search | Examiner |
|---|---|---|
| Munich | 28 February 2023 | Saunders, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.....................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 4635

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2023

| Patent document cited in search report | Publication date | Patent family member(s) | Publication date |
|---|---|---|---|
| WO 2021197927 A1 | 07-10-2021 | EP 4127288 A1<br>WO 2021197927 A1 | 08-02-2023<br>07-10-2021 |
| WO 2020254931 A1 | 24-12-2020 | NONE | |

EPO FORM P0459

For more details about this annex : see Official Journal of the European Patent Office, No. 12/82

**EP 4 299 809 A1**

REFERENCES CITED IN THE DESCRIPTION

**Patent documents cited in the description**

- WO 2021197927 A1